# EUROPEAN PATENT APPLICATION

(11) **EP 0 818 205 A2**
(43) Date of publication of application: **14.01.1998**
(21) Application number: 97304529.7
(22) Date of filing: 24.06.1997
(51) Int. Cl.: A61L 2/06, A61L 2/26

(54) **Steam sterilization**

(30) Priority: 13.07.1996 GB 9614779
(71) Applicant: Smiths Industries Public Limited Company, London, NW11 8DS (GB)
(72) Inventor: Hannant, Keith, Rustington, West Sussex BN16 2QN (GB)
(74) Representative: Flint, Jonathan McNeill

(57) **Abstract**

A sterilizer has an autoclave 1 within which is placed a sealed, non-porous bag 2 or box 50 containing an instrument 3 to be sterilized and a quantity of water or other liquid 22. When the temperature and pressure in the autoclave are raised, this raises the temperature and pressure of the container 2 and its contents sufficiently to effect sterilization, without any risk of contaminating the interior of the autoclave. The instrument 3 can be removed from the container and resterilized in the autoclave in the normal way, if desired.

## Description

This invention relates to sterilization apparatus including an autoclave chamber and means for raising the temperature and pressure within the chamber.

Conventional autoclave sterilizers have a pressure chamber and a heater for heating water and producing steam within the chamber. The steam raises the temperature and pressure within the chamber. Articles placed in the chamber are effectively sterilized as a result of the raised temperature and pressure; the increased thermal conductivity caused by the steam increases the rate of heat transfer to the articles. Because no chemical disinfectants are needed, autoclaves produce no hazardous waste and leave no harmful residues on the sterilized articles. The articles to be sterilized are usually rinsed with water before being placed in the sterilizer, so as to remove the major part of contaminated material on the article. Sometimes, the articles are placed in a porous bag after rinsing and before sterilizing, as described in US 5312250. The bag is sealed and the articles are sterilized in the bag. The porous nature of the bag material enables the steam to penetrate the bag and effect sterilization. After use, the articles are stored in the unopened bag, the pore size of the bag material being small enough to prevent the entry of bacteria and the like.

A problem exists where surgical instruments are contaminated with material that is difficult to sterilize, such as material from pathological examinations of patients suffering from CJD (Creutzfeld-Jacob disease). Instruments contaminated with such material cannot be safely rinsed before sterilization because the contaminated material may collect in waste traps and other places where it could remain hazardous because of its high resistance to conventional cleaning fluids. If, however, the instruments are placed unwashed in the sterilizer, there is also a risk of contamination. This is because the large amounts of contaminated material increases the risk that some may be released in aerosol form within the sterilizer. Although conventional filters used in the air vents of sterilizers will prevent escape of bacteria and viruses released in this way during the air venting stage of sterilization, the contaminated material may collect in more remote regions of the sterilizer, such as, adjacent the vent. These regions are not subject to the same high temperatures as the main part of the sterilizer and may contain pockets of dry air less effective than the steam at heating the contaminated material to a safe temperature. This is a particular risk in vacuum sterilizers, which pull a vacuum before beginning the sterilizing cycle. There is a risk, therefore, particularly with these forms of contaminants that require higher temperatures for effective sterilization, that parts of the sterilizer may become contaminated. This contamination may be released to subsequent sterilization loads and can also present a hazard to users and service engineers. For this reason, instruments contaminated in this way are often disposed of immediately after being used. This can be expensive and the instruments themselves can present a hazard to people handling them for disposal.

It is an object of the present invention to provide improved sterilization apparatus and methods.

According to one aspect of the present invention there is provided sterilization apparatus of the above-specified kind, characterised in that the apparatus includes a sealed, non-porous container within the chamber, that the container contains a liquid and an instrument to be sterilized, and that the apparatus is arranged to raise the temperature of the liquid in the container above its boiling point at atmospheric pressure, the increased pressure within the container being counteracted by the increased pressure in the chamber so as to prevent damage to the container.

In this way, the instrument can be sterilized without the risk of contaminated material being released into the sterilizer.

The container may be a flexible bag or a rigid box. The liquid is preferably water. The means for raising the temperature and pressure preferably includes a heating element immersed in water. The apparatus may include a vacuum pump arranged to pump air out of the chamber.

According to another aspect of the present invention there is provided a method of sterilizing an instrument comprising the steps of placing the instrument in a non-porous container containing a liquid, sealing the container, placing the container and its contents in an autoclave, subjecting the container and its contents to raised temperature and pressure sufficient to effect sterilization, and subsequently removing the instrument from the container.

The method may additionally include a subsequent step of resterilizing the instrument outside the container.

Sterilizing apparatus and a method of sterilizing, according to the present invention, will now be described, by way of example, with reference to the accompanying drawing, in which:
- Figure 1: is a schematic side elevation view of the apparatus;
- Figures 2 to 4: illustrate steps in the method of sterilizing; and
- Figure 5: is sectional side elevation view of an alternative container.

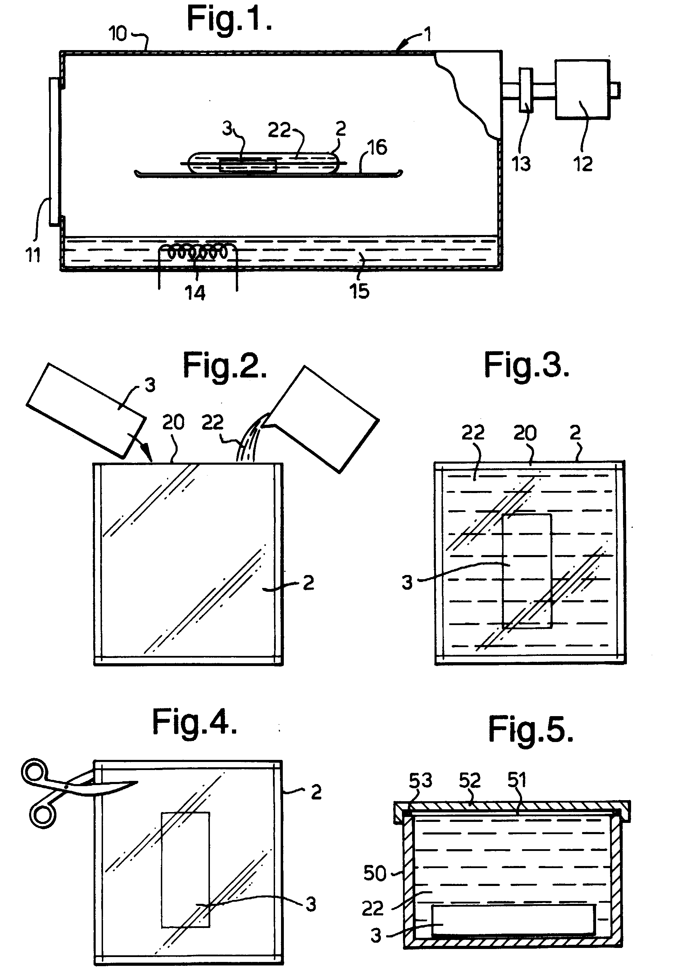

With reference to Figure 1, the apparatus comprises a conventional autoclave sterilizer 1 and a sealed container 2 containing water, in which the instrument 3 to be sterilized is placed.

The autoclave 1 has a pressure chamber 10 sealed by a door 11 at one end. A vacuum pump 12 opens into the upper end of the chamber 10 via a bacterial filter 13. Alternatively, the autoclave need not have a vacuum pump but could rely on steam pressure to drive out air through a vent before the start of the pressurizing/sterilizing phase. At the lower end of the chamber 10, a heating element 14 is immersed in water 15. The container 2 is placed in the autoclave 1, on a rack 16 above the water level. The autoclave 1 has various other conventional features, which are not relevant to an understanding of the present invention.

With reference to Figure 2, the container 2 is a flexible bag of a plastics material that is substantially impervious, that is, non-porous, to gas. The bag 2 is heat sealed around three sides and is open at its upper end 20. The instrument 3 to be sterilized, which may be unwashed, is placed in the bag 2 and this is filled with water 22 or some other liquid. The upper end 20 of the bag 2 is then sealed closed, as shown in Figure 3, such as by heat sealing. Alternatively, some form of clip or fastener could be used. The bag 2 is completely full of water 22 so that all air in the bag is excluded. The bag 2 and its contents 3 and 22 are then placed on the rack 16 in the autoclave 1 and the door 11 is closed. The vacuum pump 12 pumps down the chamber 10 to remove the air before the heating element 14 is energized to produce steam and raise the temperature and pressure in the chamber, in the usual way. The autoclave 1 undergoes a conventional sterilization cycle. The temperature in the autoclave chamber 10 raises the temperature of the water 22 in the bag 2 above its boiling point at atmospheric pressure and this heat is conducted effectively through the medium of the water to the instrument 3, so that it is thoroughly sterilized. The water 22 in the bag 2 is prevented from boiling because of the pressure exerted on it through the wall of the bag from the pressure outside the bag, in the autoclave chamber 10. The pressure on opposite sides of the walls of the bag 2 are, therefore, equalized and damage to the bag is prevented

At the end of the sterilization cycle, when the temperature and pressure in the chamber 10 have fallen to safe levels, the bag 2 and its contents 3 and 22 are removed. The bag 2 is then cut open, as shown in Figure 4, and the instrument 3 is removed. Because the instrument 3 is immersed in water 22 during the sterilization process, this helps dissolve or loosen contaminated material on the instrument. Although the bag 2 and the water 22 may be soiled, they are sterile and can be safely disposed of safely. The instrument 3 can be dried and stored or, preferably, it is washed and subject to another sterilization process, this one being a conventional sterilization. The purpose of this is to remove any traces of pyrogenic material in the sterilizing water that may remain on the instrument.

Because the contaminated instrument is completely enclosed during sterilization, there is no risk of contaminated material on the instrument contaminating the sterilizer.

The container need not be a flexible plastic bag; it could, for example, be a rigid container 50, as shown in Figure 5. This container 50 is a metal box with an open top 51 that can be closed by means of a lid 52. An O-ring seal 53 forms a gas-tight seal after closing. Again, pressure within the container 50 during sterilization is balanced by pressure outside the container, in the autoclave chamber 10. This container 50 has the advantage that it can be reused.

## Claims

1. Sterilization apparatus including an autoclave chamber and means for raising the temperature and pressure within the chamber, characterised in that the apparatus includes a sealed, non-porous container (2, 50) within the chamber (10), that the container contains a liquid (22) and an instrument (3) to be sterilized, and that the apparatus is arranged to raise the temperature of the liquid (22) in the container (2, 50) above its boiling point at atmospheric pressure, the increased pressure within the container (2, 50) being counteracted by the increased pressure in the chamber (10) so as to prevent damage to the container.

2. Sterilization apparatus according to Claim 1, characterised in that the container is a flexible bag (2).

3. Sterilization apparatus according to Claim 1, characterised in that the container is a rigid box (50).

4. Sterilization apparatus according to any one of the preceding claims, characterised in that the liquid (22) is water.

5. Sterilization apparatus according to any one of the preceding claims, characterised in that the means for raising the temperature and pressure includes a heating element (14) immersed in water.

6. Sterilization apparatus according to any one of the preceding claims, including a vacuum pump (12) arranged to pump air out of the chamber (10).

7. A method of sterilizing an instrument comprising the steps of placing the instrument (3) in a non-porous container (2, 50) containing a liquid (22), sealing the container, placing the container and its contents in an autoclave (1), subjecting the container and its contents to raised temperature and pressure sufficient to effect sterilization, and subsequently removing the instrument (3) from the container (2, 50).

8. A method according to Claim 7, including a subsequent step of resterilizing the instrument (3) outside the container (2, 50).
